# EUROPEAN PATENT APPLICATION

(11) **EP 3 375 353 A1**
(43) Date of publication of application: **19.09.2018**
(21) Application number: 17161311.0
(22) Date of filing: 16.03.2017
(51) Int. Cl.: A61B 5/00

(54) **PHOTOACOUSTIC IMAGING OF INFLAMED TISSUE**

(71) Applicant: Universität Zürich, 8006 Zürich (CH)
(72) Inventor: SCHNEIDER, Martin, 8046 Zürich (CH)
(74) Representative: Schulz, Ben Jesko

(57) **Abstract**

The present invention relates to a method, particularly for detecting inflamed tissue, like ulcerated, necrotic, granulation, fibrotic, fibrin rich-, neutrophil rich-, particularly collagen I, III, IV, V, VI rich-, or thrombin tissue in solid tissue in vivo using a pulsed laser with a wavelength in the range of e.g. 680 nm to 700nm and photon detectors which are detecting the re-emitted photons or acoustic sensors which detect the photoacoustic signal generated within the inflamed tissue. Using a pulsed laser with the wavelength 680 nm to 700nm leads to the excitation of endogenous occurring chromophores in inflamed tissue, like ulcerated, necrotic, granulation, fibrin rich-, neutrophil rich- and fibrotic tissue. A detected photoacoustic response can confirm the presence or absence of inflamed tissue. To better distinguish inflamed tissue from other naturally occurring chromophores like deoxygenated haemoglobin an additional wavelength in the range from e.g. 750nm to 770nm can be applied. Preferably, multiple photoacoustic signals are sensed from sensors that span a three dimensional space and the position of the inflamed tissue can be determined.

## Description

### Specification

The present invention relates to a method and a system for detecting inflamed tissue.

Among others, particularly such Inflamed tissue can consist out of or comprise extracellular matrix (ECM), collagen (particularly collagen I, III, IV, V, VI), fibrinogen, fibrin, plasma fibronectin, plasmin, serocellular crust, coagulated protein, stroma, basement membrane, platelets, neutrophils, macrophages, mast cells, dendritic cells, basophils, eosinophils, natural killer cells, fibroblast, myofibroblast, proteoglycan, glycoprotein (like factor VIII and V, transglutaminase (like factor XIII), elastin, matrix metalloproteinases (MMPs), fibrosis, fibrous, connective tissue, granulation tissue, prothrombin or thrombin (serine protease).

It is an objective of the present invention to provide a method for detecting inflamed tissue that is non-invasive and can particularly be performed without surgical intervention.

This problem is solved by a method having the features of claim 1 as well as by a system having the features of claim 12.

Preferred embodiments of these aspects of the present invention are stated in the sub claims and are described below.

According to claim 1, the method for detecting inflamed tissue, comprises the steps of:
- irradiating a human or animal tissue region with laser light having a wavelength in the range from 615nm to 710nm, particularly 615nm to 665 nm, preferably 680 nm to 710 nm, particularly 680nm to 700nm, in order to
   ∘ generate a Stokes fluorescence of the irradiated tissue region, wherein an emission spectrum of said Stokes fluorescence is recorded, and wherein the presence of a peak of said emission spectrum in the range from 700nm to 750nm, particularly 700 nm to 725 nm, particularly 710 nm to 725 nm is detected and particularly (e.g. automatically) attributed (e.g. by an analyzing unit, see also below) to the presence of inflamed tissue in said tissue region, and/or
   ∘ to generate a photoacoustic response signal of the irradiated tissue region comprising ultrasound waves, which are detected by at least one ultrasonic sensor (e.g. a piezoelectric sensor), which generates a corresponding output, which output is processed in order to obtain an absorption coefficient of the tissue region, and wherein the presence of a peak of said absorption coefficient is detected and particularly (e.g. automatically) attributed (e.g. by an analyzing unit, see also below) to the presence of inflamed tissue in said tissue region.

Photoacoustic imaging relies on ultrasonic detection of photoacoustically induced signals following absorption of e.g. pulsed light. The amplitude of the generated broadband ultrasound waves reflects local optical absorption properties of the irradiated tissue. Since scattering of ultrasound waves in biological tissues is extremely weak, as compared to that of light, biomedical photoacoustic imaging combines high optical absorption contrast with good spatial resolution limited only by ultrasonic diffraction.

In other words, the excitation of inflamed tissue with a laser light wavelength in the range of 615 nm to 710 nm, particularly 615 nm to 665 nm, preferably 680 nm to 710 nm, particularly 680 nm to 700 nm, leads to two surprising effects. Each effect can be detected with a particular sensor/method:
On the one hand, the generated Stokes shift is the difference (in wavelength or frequency units) between positions of the band maxima of the absorption and emission spectra (e.g. fluorescence or Raman) of the same electronic transition. When a system such as a molecule or an atom absorbs a photon it transitions to an excited state. Afterwards the system can relax by emitting a photon, thus losing the gained energy (alternatively the gained energy can be released as heat). In case the emitted photon has less energy than the absorbed photon, the resulting energy difference is denoted as Stokes shift. Stokes fluorescence is the re-emission of longer wavelength photons (i.e. lower frequency or energy) by a molecule that has absorbed photons of shorter wavelengths (i.e. higher frequency or energy). Both absorption and emission of energy are known to be unique characteristics of a particular molecular structure.

On the other hand photoacoustic imaging is a biomedical imaging technique based on the photoacoustic effect. Here, particularly non-ionizing laser light (e.g. laser pulses) is delivered into biological tissue. Some of the delivered energy will be absorbed and converted into heat, leading to transient thermoelastic expansion and thus wideband (i.e. MHz) ultrasonic emission. The generated ultrasound waves are then detected by at least one suitable ultrasonic sensor and then analyzed to produce a value of an absorption coefficient of the tissue region or an image comprised of local absorption coefficients (in case several ultrasonic sensors are used), i.e. an absorption coefficient distribution. The analyzing methods relating to photoacoustic imaging are known in the state of the art. Particularly, the methods described in US20110306857A1 may be used.

Particularly, several ultrasonic sensors are used. Then, preferably, the detected ultrasound signals are processed to obtain a distribution (i.e. a map) of local absorption coefficients of the tissue region. This distribution contains the respective local absorption coefficient for a voxel or point at a position x, y, z in the volume of the tissue region. Alternatively, also a 2D (two-dimensional) distribution may be determined.

Using several ultrasonic sensors allows one to form photoacoustic images by reconstructing the original point source of the ultrasound waves generated due to the absorption of the laser light by the tissue region. The pixel intensity in such an image can be expressed as a function of the output of the sensors (i.e. a voltage). The intensity is proportional to the absorption coefficient distribution, but also to the light fluence which in turn also depends on the absorption coefficient distribution. Methods to extract the actual absorption coefficient distribution are well-known in the state of the art and are described for instance in US20110306857.

Particularly, to obtain a photoacoustic image representing the absorption coefficient distribution, the raw data, i.e. the individual ultrasonic sensor output is backprojected which is also termed inversion. Backprojection can be conducted with the raw outputs from the sensors or with already spectrally processed outputs (see also below). Further, spectral processing can also be applied after backprojection instead. In the inversion process, a light distribution model can be used to account for the light fluence to yield a resulting distribution of absorption coefficients.

Spectral processing may particularly comprise subtraction of sensor output for two different wavelengths (see also below). For instance, in order to reduce the effect of deoxygenated haemoglobin on the final images/distributions, the tissue region may also be irradiated with (pulsed) laser light having a different further wavelength, e.g. in the region from 750 nm to 790 nm, particularly 750 nm to 780 nm, particularly 750 nm to 770 nm, where inflamed tissue shows essentially no absorption, but particularly deoxygenated haemoglobin does absorb in this region. For instance by subtracting the (particularly properly weighted) distribution for said different wavelength from the distribution for the initial wavelength, the influence of a disturbing component can be reduced.

Further, known absorption coefficient distributions of other present substances can be accounted for to get a final clean distribution/photoacoustic image of an inflamed tissue region.

Further particularly, in an embodiment, the photoacoustic response to said different further wavelength stated above (i.e. from 750 nm to 790 nm, particularly 750 nm to 780 nm, particularly 750 nm to 770 nm) is used as an input for a spectral unmixing algorithm which yields a final absorption coefficient (distribution) in which the contribution from deoxygenated haemoglobin is reduced or even eliminated.

Particularly, according to an embodiment, processing of said output of said ultrasonic sensor(s) comprises using a further output of the at least one ultrasonic sensor (or of several ultrasonic sensors) related to said irradiation of the tissue region with laser light having said further wavelength (or using a quantity derived from said further output) in a spectral unmixing algorithm to yield an absorption coefficient (or an absorption coefficient distribution) in which the contribution of deoxygenated haemoglobin is reduced or eliminated.

The specific wavelength of the laser light that is used for irradiating said tissue region to be examined and which causes said response signal/Stokes fluorescence of the tissue region in question is herein also denoted as inflamed tissue wavelength (ITW).

In this regard, the present invention is based on the surprising finding that inflamed tissue generally shows a significant response to the ITW in the Stokes fluorescence, i.e., a fluorescence emission peak located in the range from 700 nm to 750nm, particularly, 700 nm to 725 nm, particularly 710 nm 725 nm, which peak is not present in case the tissue region shows no sign of inflammation. Further, it has been surprisingly found out that also the photoacoustic response signal, i.e., an ultrasound signal emitted by the tissue region when irradiated with the ITW, particularly a (local) absorption coefficient (distribution) derived therefrom, shows a significant peak which is not present in case the examined tissue region is not inflamed.

Thus, the present invention allows to detect inflamed tissue, like ulcer-, fibrotic-, granulation-, injured or necrotic tissue by using a (pulsed) laser with an excitation wavelength that is preferably in the range from 680 nm to 710 nm, particularly 680 nm to 700 nm. The emitted signal, either photons or the thermoelastically induced ultrasonic waves can be detected by a suitable sensor device (e.g. photon sensor) or an acoustic (e.g. ultrasonic) sensor. A preferred application of the present invention is the non-invasive detection of myocarditis, pulpitis, periodontitis, peptic-, duodenal ulcers, necrotic or fibrotic tissue due to cancer or foreign bodies.

Advantageously, in the vicinity of the ITW, mammalian tissue shows reduced autofluoerescence, low light scattering and minimal absorbance of haemoglobin, melanin and water [Shcherbakova et al., 2015] which allows one to get response signals of good quality. Based on the photoacoustic effect, photoacoustic tomography (PAT) detects thermoelastically induced ultrasonic waves as a result of light absorption by biomolecules and particularly chromophores. Ultrasonic waves are scattered much less than photons in tissue. This enables PAT to be used for high resolution imaging deep in tissue. In PAT, the spatial resolution scales with the imaging depth depending on the application, while still maintaining a high depth-to-resolution ratio [Krumholz et al., 2014]

For the generation of acoustic waves, it is preferred to have the thermal expansion to be time variant. This can be achieved by using e.g. a pulsed laser according to an embodiment of the present invention or a continuous-wave (CW) laser with intensity modulation, particularly at a constant or variable frequency.

Pulsed excitations are advantageous because they provide a higher signal to noise ratio than CW excitations [Xia et al., 2014; Shcherbakova et al., 2015]. Particularly, a laser having a pulse rate of 5 Hz to 15 Hz, particularly 10 Hz can be used according to an embodiment of the present invention to illuminate the thermoelastic chromophore, other pulse rates may also be used.

Particularly, according to a further embodiment of the method according to the present invention, said ultrasound signal / waveform is detected with at least three ultrasonic sensors positioned at different locations with respect to said tissue region.

This allows one to automatically locate the position of the inflamed tissue in 3D coordinate system in a known manner. However, one ultrasonic sensor suffices to sense whether inflamed tissue is present or not.

Particularly, according to a preferred embodiment of the present invention the respective ultrasonic sensor is a piezoelectric sensor.

According to an embodiment of the present invention, detection of the respective peak described above (in the emission spectrum or in said absorption coefficients) is attributed to the presence of a biomarker, particularly one of: neutrophils, fibrinogen, fibrin, extracellular matrix (particularly at least one of collagen I, III, IV, V, VI), coagulated/degraded proteins.

Further, according to certain embodiments of the present invention, the tissue region to be tested or the inflamed tissue detected is one of or comprises on of: neutrophils, fibrinogen, fibrin, serocellular crust, coagulated/degraded protein, extracellular matrix (particularly at least one of collagen I, III, IV, V, VI), fibrotic tissue, granulation tissue, necrotic tissue (e.g. in solid tissue), a peptic ulcer, a duodenal ulcer, pulpitis and periodontitis.

Particularly, according to an embodiment, said human or animal tissue region to be tested is irradiated with said laser light in vivo.

Alternatively, said human or animal tissue region may be irradiated with said laser light ex vivo.

According to yet another embodiment, in case of generating said photoacoustic response signal said human or animal tissue region is further irradiated with laser light having a wavelength in the range from 750nm to 780, particularly 750 nm to 770 nm.

This second wavelength in the range from 750 nm to 780 nm, particularly 750 nm to 770 nm allows one to better distinguish the specific inflamed tissue response signal, from background signals like deoxygenated haemoglobin. The excitation wavelength (ITW) 615nm to 710nm, particularly 680 nm to 710 nm, particularly 680nm to 700 nm (see also above), leads to the highest absorption of inflamed tissue, whereas the excitation wavelength 750 nm to 790 nm, particularly 750 nm to 780 nm, particularly 750 nm to 770 nm, has an almost negative absorption of inflamed tissue, but a higher absorption of deoxygenated haemoglobin which allows one to distinguish the inflamed tissue signal from the deoxygenated haemoglobin signal (see also above).

Further, according to an embodiment, said irradiated human or animal tissue region is characterized by the absence of any externally applied chromophore that absorbs light in the wavelength range of said laser light (e.g. 615nm to 710nm, particularly 615 nm to 665 nm, preferably 680 nm to 710 nm, particularly 680 nm to 700 nm) that is used for irradiating the tissue region in order to generate said fluorescence / photoacoustic response.

A further aspect of the present invention is related to a system for detecting inflamed tissue according to claim 12, particularly for conducting the method according to the present invention, wherein the system comprises a laser device adapted to generate laser light having a wavelength in the range from 615nm to 710nm, particularly 615 nm to 665 nm, particularly 680 nm to 710 nm, particularly 680 nm to 700 nm (and particularly also in the range from 750 nm to 790 nm, particularly 750 nm to 780 nm, particularly 750 nm to 770 nm see above) for irradiating a human or animal tissue region, wherein the system further comprises
- a sensor device configured to record an emission spectrum of a Stokes fluorescence of the irradiated tissue region, and an analyzing unit configured to detect a peak of said emission spectrum in the range from 700 nm to 750 nm, particularly 700 nm to 725 nm, particularly 710 nm to 725 nm,
   and/or
- at least one ultrasound sensor configured to sense a photoacoustic response signal of the irradiated tissue region comprising ultrasound waves and to generate a corresponding output, and an analyzing unit (e.g. said analyzing unit) configured to obtain an absorption coefficient from said output of the at least one ultrasonic sensor and to detect a peak of said absorption coefficient.

Of course, also the features already described above in conjunction with the method according to the present invention can be used to further specify the system according to the present invention.

Furthermore, according to an embodiment of the system according to the present invention, said analyzing unit (or the respective analyzing unit) is further configured to attribute said peak of said emission spectrum to the presence of inflamed tissue in said tissue region. Alternatively or in addition, said analyzing unit (or the respective analyzing unit) is configured to attribute said peak of said absorption coefficient to the presence of inflamed tissue in said tissue region.

Further, according to an embodiment, said system comprises at least three ultrasound sensors configured to be arranged at different positions with respect to said tissue region and to sense said ultrasound waves and to generate a corresponding output, wherein the analyzing unit is configured to process said outputs of the ultrasonic sensors so as to obtain an absorption coefficient distribution of said tissue region, and wherein the presence of at least one peak of said absorption coefficient distribution is detected and particularly attributed to the presence of said biomarker or inflamed tissue at the location of said at least one peak. Due to the fact that the absorption coefficient distribution provides a map of local absorption coefficients, the location of inflamed tissue is precisely known (see also above).

Further, according to an embodiment, the laser device is further adapted to irradiate said human or animal tissue with laser light having a further wavelength in the range from 750 nm to 790 nm, 750 nm to 780 nm, particularly 750 nm to 770 nm, in order to reduce a component of the output of the respective ultrasonic sensor or of said absorption coefficient or of said absorption coefficient distribution owing to the presence of deoxygenated haemoglobin in said tissue region (see also above regarding spectral processing). Particularly, the analyzing unit is configured to conduct processing of said output of said ultrasonic sensor(s) which processing comprises using a further output of the at least one ultrasonic sensor (or of several ultrasonic sensors) related to said irradiation of the tissue region with laser light having said further wavelength (or using a quantity derived from said further output) in a spectral unmixing algorithm to yield an absorption coefficient (or an absorption coefficient distribution) in which the contribution of deoxygenated haemoglobin is reduced or eliminated.

Particularly, the analyzing unit can be formed by or comprise a computer on which a suitable software is executed that may record the response signal/Stokes fluorescence and analyze the latter with regard to determining the presence of said peaks (e.g. in the Stokes fluorescence emission spectrum or in said ultrasound signal) as well as the location of the detected inflamed tissue using information derived from ultrasound signals sensed by means of at least three ultrasonic sensors at different positions with respect to the tested tissue region.

The method and system according to the present invention can be used in an advantageous manner for examining a human or animal patient non-invasively. Using the specific inflamed tissue wave length (ITW) and corresponding sensors for detecting the respective response signal/Stokes fluorescence may render some invasive and radiative examinations redundant. Thus, significant cost savings can be achieved by using the method according to the present invention.

Preferred embodiments, further features and advantages of the present invention will be discussed below with respect to the Figures. The Figures may include schematic representations, which will be understood by artisans in view of the general knowledge in the art and the description that follows. Experimental systems will be discussed, and artisans will recognize broader features of the invention from the experimental systems and test results. Particularly,

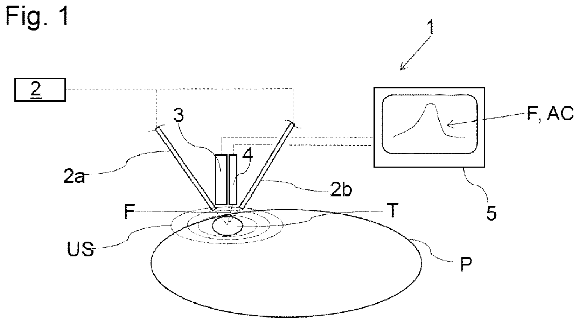

- Fig. 1: shows a schematic illustration of a set-up/system for conducting the method according to the present invention;

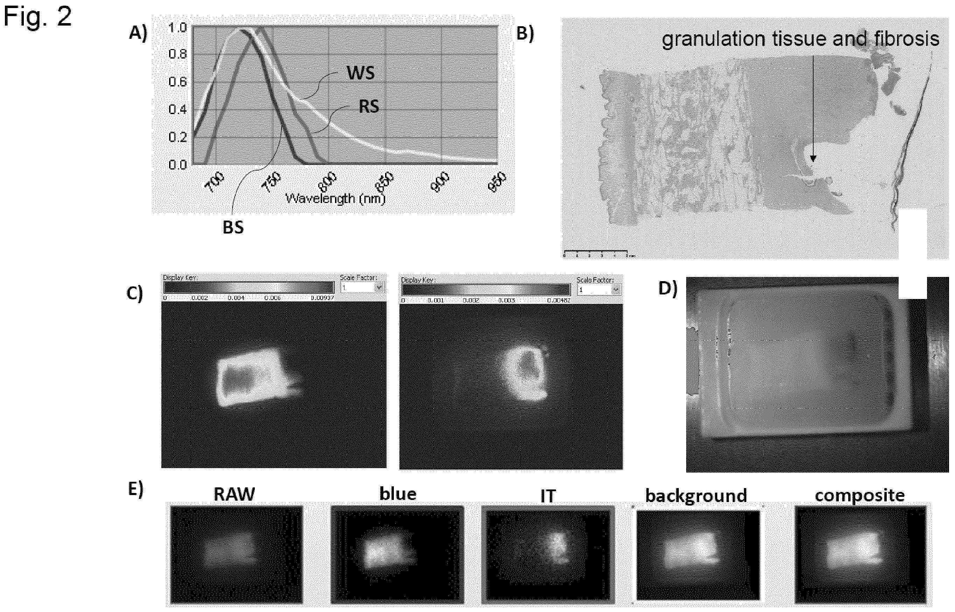

- Fig. 2: shows multispectral unmixing of granulation and fibrotic tissue in pig (ex vivo), wherein panel A) shows the fluorescent emission spectrum using the excitation wavelength of 615nm to 665nm (ITW) with an emission filter (700nm) cut-in, which lets wavelengths above 700nm pass the emission filter, wherein the white line WS shows the spectrum of the auto-fluorescence/background, the blue line BS shows the estimated spectrum of normal tissue and the red line RS shows the estimated spectrum for the granulation tissue. Further, panel B) shows hematoxylin and eosin (H&E) staining. Further, panel C) shows the fluorescence intensity of the normal tissue and the inflamed tissue (from left to right) irradiated with the inflamed tissue wavelength (ITW) 615nm to 665nm, wherein the unmixed blue fluorescence signal BS (panel A)) shows a higher fluorescence intensity in the healthy tissue, whereas the unmixed inflamed tissue (IT) fluorescence signal (red line RS of panel A)) shows a higher fluorescence intensity in the granulation tissue and fibrosis. Further, panel D) shows the irradiated tissue embedded in paraffin. Further, panel E) shows an overview of raw and unmixed fluorescence images, consisting (from left to right) of the raw fluorescence image; unmixed blue signal BS (normal tissue); unmixed IT signal RS (i.e. fluorescence response of inflamed tissue to irradiation with ITW); unmixed background signal WS; composition of blue signal BS and IT signal RS.

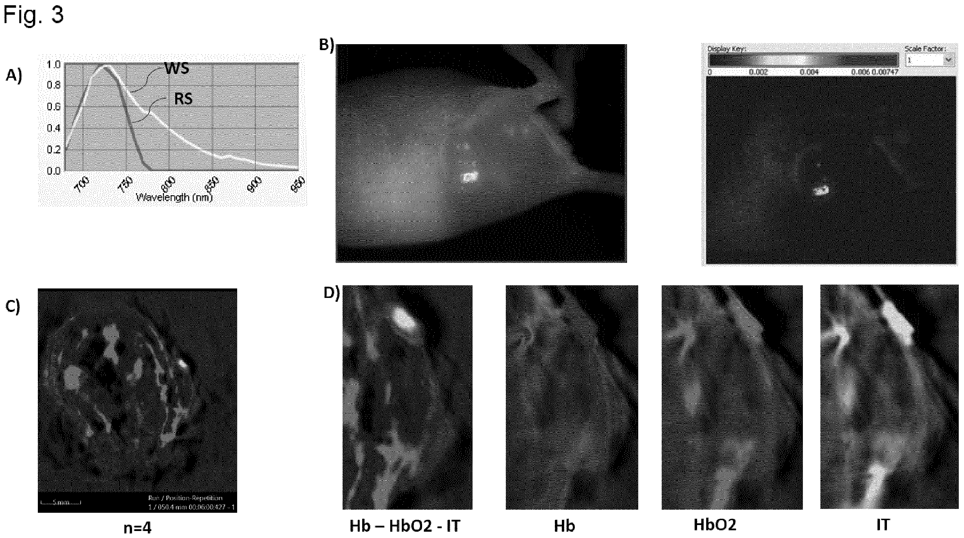

- Fig. 3: shows multispectral unmixing of wound/ulcerated/necrotic tissue in a living mouse (in vivo), wherein panel A) shows a fluorescent emission spectrum using an excitation wavelength of 615nm to 665nm (ITW) with an emission filter of 700nm cut-in and an exposure time of 2487ms. The white line WS shows the background/autofluorescence and the red line RS shows the spectrum of the inflamed/necrotic area (IT fluorescence signal). Further, panel B) shows (from left to right) the unmixed fluorescence image containing the background/autofluoerescence signal and the IT fluorescence signal (inflamed/necrotic tissue) as well as a fluorescence intensity image of the IT fluorescence signal. Further, panel C) shows the overview of multispectral unmixing of photoacoustic signals of deoxygenated haemoglobin (Hb), oxygenated haemoglobin (HbO2) and inflamed/necrotic tissue (IT) overlayed on a 900 nm single wavelength anatomical image from a mouse with ulcerative/necrotic area. The photoacoustic images were acquired with multispectral photoacoustic imaging also denoted as multispectral opto-acoustic tomography (MSOT) using an inVision 128 (iThera, Munich). The photoacoustic signals were acquired in the range from 680nm to 900nm in 10nm steps. However, post-acquisition calculations revealed that the use of the ITW (e.g. 680nm to 700nm) and particularly of a further wavelength in the range of e.g. 750nm to 780nm is enough to distinguish the IT-signal from any other naturally occurring biomolecule/chromophore like deoxygenated haemoglobin. Further, panel D) shows the magnification of panel C), namely a 900 nm single wavelength MSOT image (grayscale) with an overlay (Hb, HbO2, IT) of the spectrally-resolved deoxygenated haemoglobin, oxygenated haemoglobin and IT-signal, further a 900 nm single wavelength MSOT image (grayscale) with an overlay (Hb) of the spectrally-resolved deoxygenated haemoglobin signal, as well as a 900 nm single wavelength MSOT image (grayscale) with an overlay (HbO2) of the spectrally-resolved oxygenated haemoglobin signal, and finally a 900 nm single wavelength MSOT image (grayscale) with an overlay (IT) of the spectrally-resolved IT-signal (the MSOT images at 900nm are used to get an anatomical picture in order to assign the IT signal to the respective anatomy).

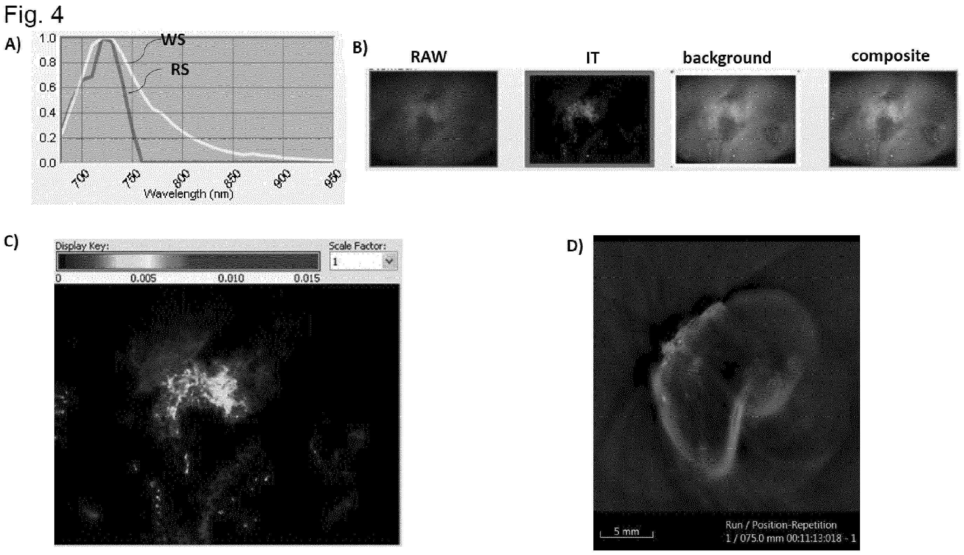

- Fig. 4: shows multispectral unmixing of peptic ulcer in horse ex vivo, wherein panel A) shows a fluorescent emission spectrum using an excitation wavelength of 615nm to 665nm (ITW) with the emission filter (700nm) cutin and an exposure time of 700ms. The white line WS shows the background/autofluorescence signal while the red line RS shows the spectrum of the inflamed tissue (IT signal). Further, panel B) shows an overview of the different fluorescence image components, consisting of (from left to right) the raw image; unmixed IT signal; unmixed background/autofluorescence signal; composition of IT signal and background/autofluorescence signal. Further, panel C) shows a fluorescence intensity of the IT fluorescence signal, and panel D) shows the overview of multispectral unmixing of photoacoustic signals of deoxygenated haemoglobin (Hb), oxygenated haemoglobin (HbO2) and inflamed/necrotic tissue (IT) overlayed on a 900 nm single wavelength "anatomical" image from a peptic ulcer in horse (ex vivo). The photoacoustic images were acquired with multispectral opto-acoustic tomography (MSOT) using an inVision 128 (iThera, Munich). The photoacoustic signals were acquired in the range from 680nm to 900nm in 10nm steps. Post-acquisition calculations revealed that the use of the ITW (e.g. 680nm to 700nm) and particularly of the further wavelength in the range from e.g. 750nm to 780nm is enough to distinguish the IT-signal from any other naturally occurring biomolecule/chromophore like deoxygenated haemoglobin.

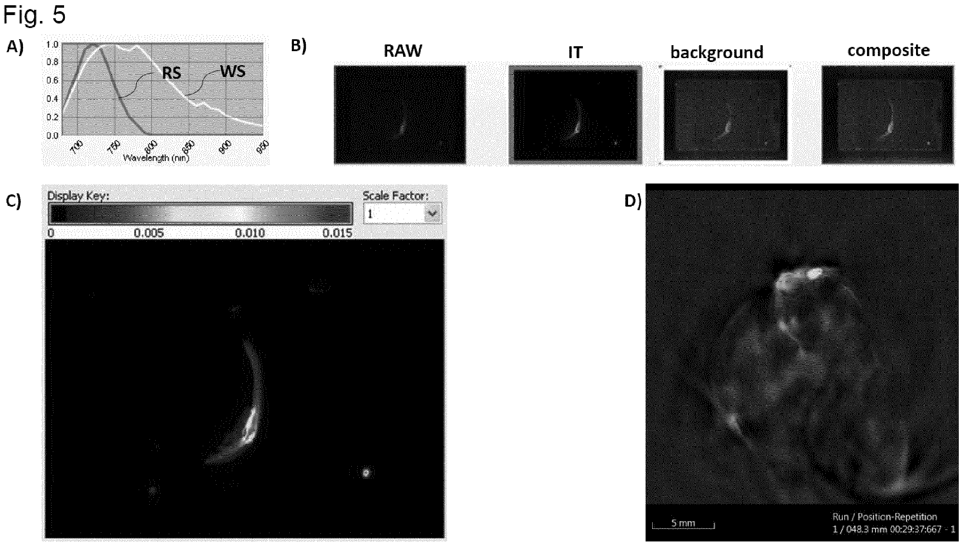

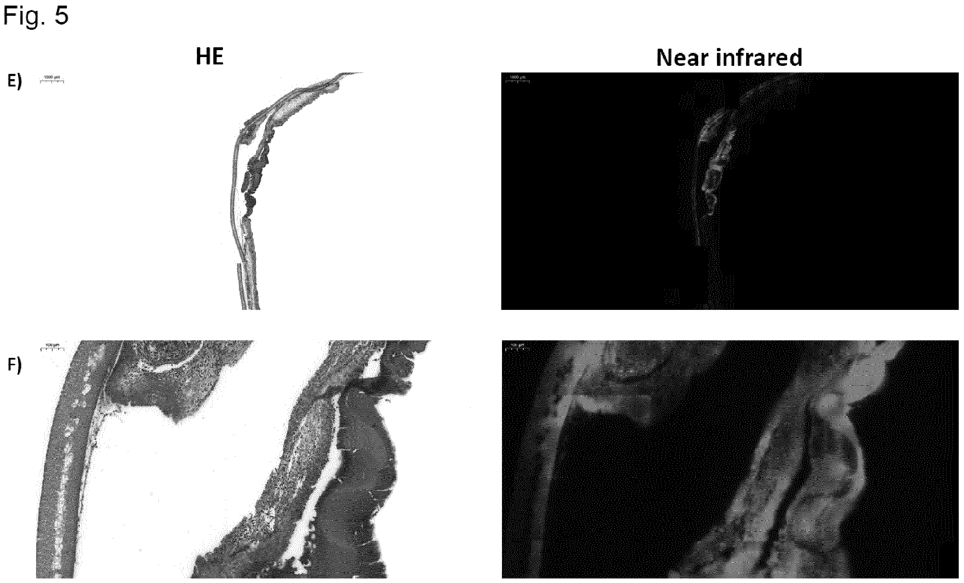

- Fig. 5: shows multispectral unmixing of a 3 day wound in mouse (ex vivo), wherein panel A) shows a fluorescent emission spectrum using an excitation wavelength of 615nm to 665nm (ITW) with emission filter (700 nm) cut-in and an exposure time of 3186ms. The white line WS shows the background/autofluorescence signal, and the red line RS shows the spectrum of the inflamed tissue (IT- signal). Further, panel B) shows an overview of the different fluorescence image components, consisting of (from left to right) the raw image; unmixed IT signal; unmixed background/autofluorescence signal; composition of IT signal and background/autofluoerescence signal. Further, panel C) shows a fluorescence intensity of the IT fluorescence signal. Further, panel D) shows the overview of multispectral unmixing of photoacoustic signals of inflamed/wound tissue overlayed on a 900 nm single wavelength "anatomical" image from a 3 day wound in mouse (ex vivo). The photoacoustic images were acquired with multispectral photoacoustic imaging (i.e. multispectral opto-acoustic tomography (MSOT) using an inVision 128 (iThera, Munich)). The photoacoustic signals were acquired in the range of 680nm to 900nm in 10nm steps. Post-acquisition calculations revealed that the use of the ITW (680nm to 700nm) and particularly of a further wavelength in the range from e.g. 750nm to 780nm for exciting the tissue is enough to distinguish the IT-signal from any other naturally occurring biomolecule/chromophore like deoxygenated haemoglobin. Further, panel E) shows the histology of a 3 day wound in mouse (ex vivo), wherein the left hand side shows hematoxylin and eosin (H&E) staining and the right hand side shows fluorescent imaging using the excitation wavelength 633nm and an emission filter 700/75. Further, panel F) shows a magnification (10x) of the wound area, and

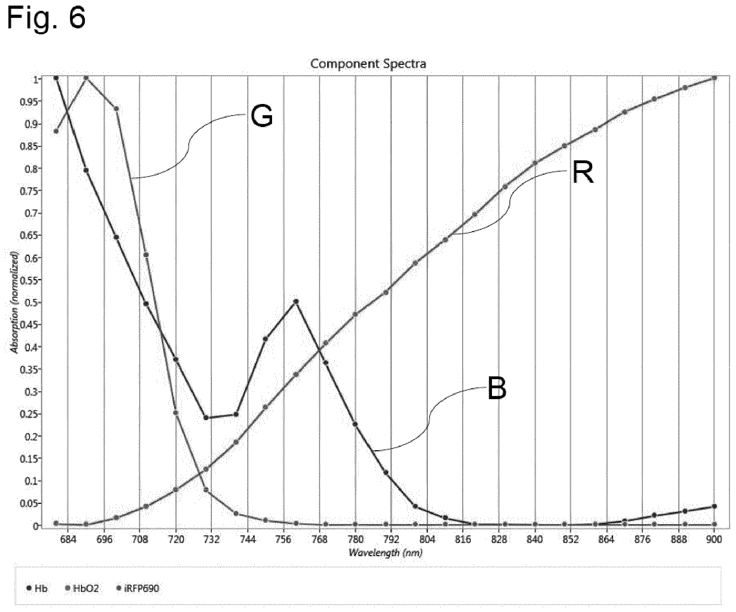

- Fig. 6: shows photoacoustic absorption spectra of Hb (reference numeral B), HbO₂ (reference numeral R), and iRFP690 (reference numeral G).

Fig. 1 shows a schematic and exemplary representation of a system 1 for conducting the method according to the present invention. Alternative set-ups may also be used.

The system 1 comprises a laser 2 for irradiating a human or animal tissue region T of a human/animal patient P with laser light having a wavelength in the range from 615 nm to 710 nm, particularly 680nm to 710nm, particularly 680 nm to 700 nm in order to generate a Stokes fluorescence F (or a photoacoustic response US) of the irradiated tissue region T, wherein an emission spectrum of said Stokes fluorescence is recorded using one or several suitable light sensors 4, and wherein the presence of a peak of said emission spectrum in the range from 690 nm to 750 nm, particularly 700nm to 750nm, particularly 700 nm to 725 nm, particularly 710 nm to 725 nm is detected and particularly attributed to the presence of inflamed tissue in said tissue region T or one of the biomarkers stated above. Alternatively or in addition, a photoacoustic response signal US of the irradiated tissue region T in the form of ultrasound waves US is excited/analyzed using said laser light with said wavelength, which ultrasound waves US are detected by at least one ultrasonic sensor (e.g. a piezoelectric sensor) 3 and a photoacoustic image indicative of absorption coefficients AC of the tissue region is derived (the image is a constant in case of a single sensor), wherein a certain height or peak of the absorption coefficient is particularly attributed to the presence of inflamed tissue in said tissue region. Here, such a height or peak is considered to be present when the signal surpasses a standard threshold. Such a threshold may be defined using a calibration procedure, i.e., by exciting healthy tissue that is not inflamed.

The present invention thus uses the natural optical absorption of inflamed tissue, which is particularly characterized through protein degradation, granulated, fibrotic- and extracellular remodeling processes (particularly one of collagen I, III, IV, V, VI). The optical absorption of inflamed tissue is utilized to generate the necessary photoacoustic response US for detection.

The laser light can be delivered to the tissue region via optical fibers 2a, 2b. Other ways are also conceivable. Further, instead of a single ultrasound sensor 3 also a plurality of such sensors 3 can be used in order to also locate the spatial position of the detected inflamed tissue (see also below) by determining an absorption coefficient distribution of the tissue region that reveals the individual local absorption coefficients for each voxel/pixel of a rendered volume of the tissue region. For instance, the sensor 3 can be an array of ultrasonic sensors (e.g. piezoelectric sensors) as e.g. used in ultrasound probe heads.

Furthermore, a space between the tissue region T and the sensors 3 is preferably be filled with an acoustic medium such as water or a suitable gel for detecting the photoacoustic response. The tissue region T may also be embedded in such a medium.

The excitation of the Stokes fluorescence F and the photoacoustic response US can be done in a successive fashion.

For analyzing the acoustic response (ultrasound waves) US and/or the Stokes fluorescence F, the system comprises an analyzing unit 5 which may be formed by a computer on which a suitable software is executed. The analyzing unit 5 / computer 5 then determines the presence or absence of inflamed tissue from the Stokes response F and/or the presence or absence of inflamed tissue, and preferably the position of the inflamed tissue, from the processed output of the sensors 3. Any inflamed tissue in the tissue region T is revealed by a spectrum of distinct acoustic waves US sensed by the ultrasonic sensor(s) 3 and analyzed by the analyzing unit / computer 5.

The invention thus provides a method for photoacoustic/optical detection of inflamed tissue, like collagen-rich (particularly one of collagen I, III, IV, V, VI), neutrophil rich-, fibrin rich-, fibrotic-, granulation-, ulcerated, necrotic tissue in solid tissue in vivo and in vitro / ex vivo. Particularly, the method is capable of detecting and geographically locating inflamed tissue. Preferred embodiments/applications of method according to the present invention is the detection of inflamed tissue in the human body, particularly peptic-, duodenal ulcers. The analyte in that case is the peptic ulcer itself.

In other preferred embodiments, other types of inflamed tissue occurring for example in fat necrosis of the breast, differentiation between glioma and radiation necrosis, bisphosphonate-related osteonecrosis of the jaw, Crohn's disease, ulcerative colitis, gangrenous cholecystitis, myocarditis, pulpitis and periodontitis can be detected.

### Examples

Particularly, Fig. 2, 3, 4, 5, shows the specific emission spectrum of ulcerative-, necrotic-, granulation- and wound tissue (inflamed tissue) acquired with the excitation wavelength 615nm to 665nm with emission filter (700 nm) cut-in. It shows the clear absorption of that wavelength and the resulting specific emission spectrum with the Stoke shift to a peak in the emission spectrum (Stokes fluorescence F) at 710 nm to 740 nm. This specific wavelength and the resulting intensity imaging of the sample correlates well with ulcerative-, necrotic-, granulation- and wound tissue. Additionally, Fig. 2 and Fig. 5 show the correlation between the detected fluorescence intensity of the IT signal and the corresponding HE staining (see also above).

Further in Figs. 3, 4 and 5, a specific photoacoustic response spectrum US from inflamed, ulcerative, necrotic, wound skin was unambiguously detected and information was obtained from multiple ultrasound sensors 3 (here a 270 degree tomographic array of an inVision 128) which allowed the determination of the location of the inflamed/ulcerative/necrotic skin in the whole mouse (ex vivo and in vivo) and in peptic ulcer from horse (ex vivo). Normal/healthy skin tissue and any other normal/healthy tissue in whole mouse body showed no photoacoustic spectrum specific response. The unique photoacoustic spectrum of inflamed tissue can be obtained by applying the ITW using e.g. the excitation wavelengths in the range of e.g. 680nm to 700nm and particularly a further wavelength in the range from e.g. 750nm to 780nm. Thus the detection method can be used to detect the presence of inflamed/ulcerative/necrotic tissue (and in general their components) in vivo. It can also be used to guide histological biopsies of inflamed tissue, increasing the accuracy of a significant biopsy.

The mouse, when put in place, receives laser radiation from two circular arranged rows of five fiber optic arrays each. Both circles are placed axially apart. The fiber optic arrays emit the laser radiation towards the centers of the circles onto the small animal.

The laser radiation is e.g. emitted in very short bursts of laser energy of 8 ns duration, 10 mJ radiant energy per fiber optic array at a rate of ten bursts per second. The pulse energy differs at different wavelengths. In the range from 700 nm 900nm it can be in the range from 80 mJ to 120 mJ.

The wavelength is set by the user within the range from 680 nm (red, visible) to 900 nm (infrared, invisible). Anatomical images between 850nm-900nm offer a compromise between light penetration into tissue, laser performance and low absorption from water, which increases significantly above 900nm. From this data the radiant power can be calculated, which amounts to 1.25 Megawatt.

One technique to determine the location of the inflamed/necrotic/ulcerative tissue within the body via determination of the absorption coefficient distribution is photoacoustic backprojection of the output of the sensors 3, which can therefore be used to detect the presence and location of inflamed tissue and to guide a histological examination. After the detection of inflamed tissue the physician can decide which procedure should succeed. Backprojection is a mathematical process that is similar to triangulating a signal using different locations. A suitable backprojection technique is disclosed in "Iterative Reconstruction Algorithm for Optoacoustic Imaging,"(Paltauf et al., 2002)

After backprojection of the sensor output - which allows efficient generation of anatomical images - spectral processing may be applied. Here one or several known spectra can be used to extract a photoacoustic image in which the influence of unwanted substances (corresponding to known spectra) is reduced.

To distinguish inflamed/necrotic/ulcerative tissue from normal tissue the spectrum of iRFP 690 can be applied in such a processing which surprisingly shows a spectrum that is very similar to that of inflamed tissue. For detecting the unique IT-spectra (iRFP 690) it is enough to irradiate the tissue region T with the ITW wavelength regions stated herein, namely particularly 680 nm to 710 nm, particularly 680 nm to 700 nm, and particularly one of said further wavelengths in the range from e.g. 740 nm to 790nm (particularly 750 nm to 770nm), see also above. Additionally one can measure/irradiate at 800nm.

The increased light absorption of inflamed/necrotic/ulcerative tissue in the excitation wavelength of e.g. 680 nm to 710nm and the low light absorption between e.g. 740 nm to 790nm are unique for inflamed/necrotic/ulcerative tissue and can be therefore discriminated from other natural occurring chromophores like deoxygenated haemoglobin - with a higher light absorption at 740 nm to 790 nm.

Particularly, the excitation wavelength of e.g. 680 nm to 710 nm leads to the highest absorption of inflamed tissue and the excitation wavelength of e.g. 750nm to 770 nm has an almost negative absorption of inflamed tissue, but a higher absorption of deoxygenated haemoglobin (cf. Fig. 6) which is important to distinguish the inflamed tissue signal from the deoxygenated haemoglobin signal.

A similar technique with different wavelengths has been described by Viator, et. Al. "Photoacoustic Discrimination of Viable and Thermally Coagulated Blood Using a Two-Wavelength Method for Burn Injury Monitoring" (Talbert et al., 2007). Here it is shown that two wavelengths can be used to distinguish between blood and melanin. The unique absorption spectrum of haemoglobin in contrast to the simple spectrum of melanin makes such a classification possible.

### References

Krumholz, A., Shcherbakova, D. M., Xia, J., Wang, L. V., and Verkhusha, V. V. (2014). Multicontrast photoacoustic in vivo imaging using near-infrared fluorescent proteins. Scientific reports 4, 3939.
Paltauf, G., Viator, J. A., Prahl, S. A., and Jacques, S. L. (2002). Iterative reconstruction algorithm for optoacoustic imaging. The Journal of the Acoustical Society of America 112, 1536-1544.
Shcherbakova, D. M., Baloban, M., and Verkhusha, V. V. (2015). Near-infrared fluorescent proteins engineered from bacterial phytochromes. Current opinion in chemical biology 27, 52-63.
Talbert, R. J., Holan, S. H., and Viator, J. A. (2007). Photoacoustic discrimination of viable and thermally coagulated blood using a two-wavelength method for burn injury monitoring. Physics in medicine and biology 52, 1815-1829.
Xia, J., Yao, J., and Wang, L. V. (2014). Photoacoustic tomography: principles and advances. Electromagnetic waves 147, 1-22.

## Claims

1. Method for detecting a fluorescence and/or photoacoustic response, particularly for detecting inflamed tissue, comprising the steps of:
- irradiating a human or animal tissue region (T) with laser light having a wavelength in the range from 615 nm to 710 nm in order to
∘ generate a Stokes fluorescence (F) of the irradiated tissue region (T), wherein an emission spectrum of said Stokes fluorescence is recorded, and wherein the presence of a peak of said emission spectrum in the range from 700 nm to 750 nm is detected,
and/or
∘ to generate a photoacoustic response (US) of the irradiated tissue region (T) comprising ultrasound waves, which are detected by at least one ultrasonic sensor (3), wherein a corresponding output of the at least one ultrasonic sensor is processed in order to obtain an absorption coefficient of said tissue region, and wherein the presence of a peak of said absorption coefficient is detected.

2. The method according to claim 1, **wherein** said laser light irradiating said human or animal tissue comprises a wavelength in the range from 680 nm to 700 nm.

3. The method according to claim 1 or 2, **wherein** detection of the respective peak is attributed to the presence of a biomarker, particularly one of: neutrophils, fibrin, extracellular matrix, collagen I, collagen III, collagen IV, collagen V, collagen VI, coagulated/degraded proteins.

4. The method according to one of the preceding claims, **wherein** detection of the respective peak is attributed to the presence of inflamed tissue in said tissue region (T).

5. The method according to one of the preceding claims, **wherein** said ultrasound waves (US) are detected with at least three ultrasonic sensors (3) positioned at different locations with respect to said tissue region (T), wherein corresponding output of said ultrasonic sensors is processed in order to obtain an absorption coefficient distribution of said tissue region, and wherein the presence of at least one peak of said absorption coefficient distribution is detected.

6. The method according to one of the preceding claims, **wherein** said human or animal tissue region is further irradiated with laser light having a wavelength in the range from 750 nm to 770 nm in order to reduce a component of the output of the respective ultrasonic sensor or of said absorption coefficient or of said absorption coefficient distribution owing to the presence of deoxygenated haemoglobin in said tissue region.

7. The method according to one of the preceding claims, **wherein** said irradiated human or animal tissue region is **characterized by** the absence of any externally applied chromophore that absorbs light in the wavelength range of said laser light.

8. The method according to one of the preceding claims, **wherein** the laser light is pulsed laser light, wherein particularly the laser light has a pulse rate in the range from 5 Hz to 15 Hz.

9. The method according to one of the claims 1 to 7, **wherein** the laser light is continuous-wave intensity modulated laser light.

10. The method according to one of the preceding claims, **wherein** the inflamed tissue is one of or comprises one of: fibrotic tissue, granulation tissue, necrotic tissue, a peptic ulcer a duodenal ulcer, neutrophil rich tissue, fibrin rich tissue, collagen rich tissue, collagen I, collagen III, collagen IV, collagen V, collagen VI; wound crust.

11. The method according to one of the preceding claims, **wherein** said human or animal tissue region (T) is irradiated with said laser light in vivo or ex vivo, particularly in vitro.

12. A System (1) for detecting inflamed tissue, particularly for conducting the method according to one of the preceding claims, wherein the system (1) comprises a laser device (2) adapted to generate laser light having a wavelength in the range from 615 nm to 710 nm, particularly 680 nm to 700 nm, for irradiating a human or animal tissue region (T), wherein the system (1) further comprises
- a sensor device (4) configured to record an emission spectrum of a Stokes fluorescence (F) of the irradiated tissue region, and an analyzing unit (5) configured to detect a peak of said emission spectrum in the range from 700 nm to 750 nm,
and/or
- at least one ultrasound sensor (3) configured to sense a photoacoustic response signal (US) of the irradiated tissue region (T) in the form of ultrasound waves and to generate a corresponding output, and an analyzing unit (5) configured to obtain an absorption coefficient from a said output of the at least one ultrasonic sensor and to detect a peak of said absorption coefficient.

13. The system according to claim 12, wherein said analyzing unit (5) is further configured to attribute said peak of said emission spectrum to the presence of inflamed tissue in said tissue region (T) and/or wherein said analyzing unit (5) is further configured to attribute said peak of said absorption coefficient to the presence of inflamed tissue in said tissue region (T).

14. The system according to claim 12 or 13, wherein said system (1) comprises at least three ultrasonic sensors (3) configured to be arranged at different positions with respect to said tissue region (T) and to sense said ultrasound waves and to generate a corresponding output, wherein the analyzing unit (5) is configured to process said outputs so as to obtain an absorption coefficient distribution of said tissue region, and wherein the presence of at least one peak of said absorption coefficient distribution is detected, and particularly attributed to the presence of said biomarker or inflamed tissue at the location of said at least one peak.

15. The system according to one of the claims 12 to 14, **wherein** the laser device is further adapted to irradiate said human or animal tissue with laser light having a wavelength in the range from 750 nm to 770 nm in order to reduce a component of the output of the respective ultrasonic sensor or of said absorption coefficient or of said absorption coefficient distribution owing to the presence of deoxygenated haemoglobin in said tissue region.
